# EUROPEAN PATENT APPLICATION

(11) **EP 4 276 165 A1**
(43) Date of publication of application: **15.11.2023**
(21) Application number: 22194312.9
(22) Date of filing: 07.09.2022
(51) Int. Cl.: C12M 1/00

(54) **MICROALGAE REACTION UNIT USING WAVELENGTH CONVERSION AND MICROALGAE CULTURE SYSTEM USING THE SAME**

(30) Priority: 09.05.2022 KR 20220056571
(71) Applicant: Sherpa Space Inc., Daejeon 34028 (KR)
(72) Inventor: YUN, Choa Mun, Daejeon (KR)
(74) Representative: Schmitt-Nilson Schraud Waibel Wohlfrom Patentanwälte Partnerschaft mbB

(57) **Abstract**

Disclosed is a microalgae culture system using wavelength conversion which selectively provides light having a predetermined wavelength band depending on the species, the growth step and the health status of microalgae received in a reaction unit and a target material to be produced from the microalgae, so as to increase growth efficiency of the microalgae or to improve the output of the target material to be produced from the microalgae. The microalgae culture system includes a first culture sector including microalgae reaction units using a wavelength conversion material configured to transmit light of a first wavelength, a second culture sector including microalgae reaction units using a wavelength conversion material configured to transmit light of a second wavelength, and a control pump configured to transfer microalgae in the first culture sector to the second culture sector through a transmission pipe, when a control condition is achieved.

## Description

### BACKGROUND OF THE INVENTION

### Field of the Invention

The present invention relates to microalgae culturing technology using wavelength conversion, and more particularly, to a microalgae reaction unit which selectively provides light having a predetermined wavelength band depending on the species, the growth step and the health status of microalgae received in the reaction unit and a target material to be produced from the microalgae, so as to increase growth efficiency of the microalgae or to improve the output of the target material to be produced from the microalgae, and a microalgae culture system using the microalgae reaction unit.

### Description of the Related Art

Recently, interest in photosynthetic microorganisms or microalgae picks up due to functional diversity thereof, and the range of research on these microorganisms or microalgae are being widened in various fields. Microalgae having photosynthetic capacity is being actively applied to research on reduction of carbon dioxide which is spotlighted now due to environmental problems, such as global warming, and is being also applied to research regarding production of bioenergy, such as biodiesel, bioethanol and hydrogen gas, which are spotlighted as sustainable energy sources in preparation for depletion of fossil fuels.

However, in order to achieve quantitively significant removal of carbon dioxide and mass-production of useful products, such as bioenergy, using microalgae, the microalgae should be cultured on a large scale and in a large concentration. Therefore, technology relating to construction of large-scale culturing facilities is required.

Particularly, in terms of microalgae culture, microalgae should be cultured in consideration of a light wavelength and a light intensity, and thus, the light wavelength is more important than anything else. The concentration of the microalgae and the concentration of metabolites produced from the microalgae may be improved by supplying a specific light wavelength or range to the microalgae.

However, an apparatus, which is provided in a microalgae culture apparatus so as to generate a light wavelength, occupies the largest proportion of production costs, and is thus disadvantageous in terms of initial installation costs and maintenance.

### RELATED ART DOCUMENT

### PATENT DOCUMENT

Korean Patent Registration No. 10-1188745 (September 28, 2012)

### SUMMARY OF THE INVENTION

Therefore, the present invention has been made in view of the above problems, and it is an object of the present invention to provide a microalgae culture system using wavelength conversion which selectively provides light having a predetermined wavelength band depending on the species, the growth step and the health status of microalgae received in a reaction unit and a target material to be produced from the microalgae, so as to increase growth efficiency of the microalgae or to improve the output of the target material to be produced from the microalgae.

In accordance with an aspect of the present invention, the above and other objects can be accomplished by the provision of a microalgae culture system using wavelength conversion, including a first culture sector including microalgae reaction units using a wavelength conversion material configured to transmit light of a first wavelength, a second culture sector including microalgae reaction units using a wavelength conversion material configured to transmit light of a second wavelength, and a control pump configured to transfer microalgae in the first culture sector to the second culture sector through a transmission pipe, when a control condition is achieved.

The first wavelength and the second wavelength may be determined depending on at least one of a species of the microalgae corresponding to a target to be cultured, a growth step of the microalgae, a health status of the microalgae, or a target material to be produced from the microalgae.

The microalgae reaction units may be closed-type units having any one of a plate type, a column type, an annular type, a tubular type, and a sack type.

Each of the microalgae reaction units may include a light-transmitting shape layer, and a wavelength conversion layer including a wavelength conversion material configured to transmit light of a predetermined wavelength band.

The wavelength conversion layer may include a flexible film layer including the wavelength conversion material, and a bonding layer having adhesiveness to be adhered to the shape layer.

The wavelength conversion layer may be formed by applying the wavelength conversion material to the shape layer.

The microalgae reaction units may be manufactured by performing molding of a mixture of a light-transmitting shape material and a wavelength conversion material configured to transmit light of a predetermined wavelength band.

The microalgae culture system using wavelength conversion may further include at least one of cameras configured to capture images of the microalgae reaction units, optical sensors configured to sense light transmittances of the microalgae reaction units, or temperature sensors configured to measure inner temperatures of the microalgae reaction units, and a controller configured to determine cultured states of the microalgae in the microalgae reaction units based on at least one of the images captured by the cameras, the light transmittances, or the inner temperatures, and to transmit light of an order to transfer the microalgae to the control pump based on input of manager's instructions or an automated algorithm when the determined cultured states of the microalgae conform to a predetermined standard.

The wavelength conversion material may include at least one of an inorganic phosphor, quantum dots or perovskite.

In accordance with another aspect of the present invention, there is provided a microalgae reaction unit using wavelength conversion, configured to employ a wavelength conversion material configured to selectively transmit light of a predetermined wavelength band depending on at least one of a species microalgae corresponding to a target to be cultured, a growth step of the microalgae, a health status of the microalgae, or a target material to be produced from the microalgae.

The microalgae reaction unit using wavelength conversion may be a closed-type unit having any one of a plate type, a column type, an annular type, a tubular type, and a sack type.

The microalgae reaction unit using wavelength conversion may include a light-transmitting shape layer, and a wavelength conversion layer including a wavelength conversion material configured to transmit light of a predetermined wavelength band.

The wavelength conversion layer may include a flexible film layer including the wavelength conversion material, and a bonding layer having adhesiveness to be adhered to the shape layer.

The wavelength conversion layer may be formed by applying the wavelength conversion material to the shape layer.

The microalgae culture unit using wavelength conversion may be manufactured by performing molding of a mixture of a light-transmitting shape material and a wavelength conversion material configured to transmit light of a predetermined wavelength band.

The wavelength conversion material may include at least one of an inorganic phosphor, quantum dots or perovskite.

### BRIEF DESCRIPTION OF THE DRAWINGS

The above and other objects, features and other advantages of the present invention will be more clearly understood from the following detailed description taken in conjunction with the accompanying drawings, in which:
FIG. 1 is a view illustrating relations between growth of microalgae and a light wavelength;
FIGs. 2(A) to 2(D) are views showing types of reaction units in which microalgae are cultured, according to one embodiment of the present invention;
FIGs. 3 to 5 are perspective views showing the respective types of the reaction units exemplarily shown in FIGs. 2(A) to 2(D);
FIG. 6 is a schematic view illustrating a microalgae culture system using wavelength conversion according to one embodiment of the present invention;
FIG. 7 is a view showing a modified example of culture sectors shown in FIG. 6;
FIG. 8 is a view showing light wavelength bands provided in order of the growth steps of the microalgae in the system according to the present invention; and
FIGs. 9 to 12 are graphs showing optical spectra of the light wavelength bands provided in the respective growth steps;
FIG. 13 is a graph representing relations between growth of the microalgae and production of astaxanthin depending on a light wavelength band; and
FIG. 14 is a schematic view illustrating a microalgae culture system using wavelength conversion according to another embodiment of the present invention.

### DETAILED DESCRIPTION OF THE INVENTION

Hereinafter reference will be made in detail to exemplary embodiments of the present invention, examples of which are illustrated in the accompanying drawings. However, it will be understood that the embodiments of the present invention are provided only to completely disclose the invention and cover transformations, equivalents or substitutions which come within the scope and technical range of the invention.

In the following description of the embodiments, singular forms may be intended to include plural forms as well, unless the context clearly indicates otherwise.

In the following description of the embodiments, it will be understood that, when a part "has" or "comprises" an element, the part may further include other elements, and does not exclude the presence of such other elements, unless stated otherwise.

In the following description of the embodiments, it will be understood that the terms "...unit", "...module" and "...component" indicate units for processing at least one function or operation, and may be implemented using hardware, software or a combination of hardware and software.

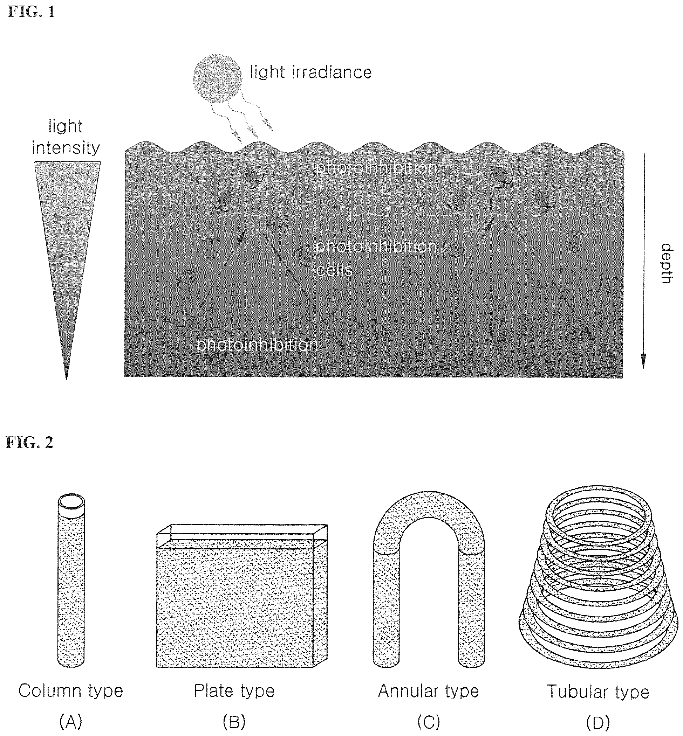

FIG. 1 is a view illustrating relations between growth of microalgae and a light wavelength.

A light intensity (or a light wavelength) is one of main limiting factors in microalgae culture, and the duration and strength of a light source directly influence photosynthesis of microalgae, and further influence the output of biomass.

The light wavelength used in growth of microalgae are varied depending on the species or the growth step of the microalgae, because a photoreceptor varies depending on a metabolic pathway, pigmentation, the species and the growth step of the microalgae.

In order to prevent photooxidation and inhibition of growth of microalgae, a bioreactor for microalgae requires a proper light intensity and proper light duration. Microalgae are not capable of being effectively grown at an excessively high or low light intensity.

Various light intensities are applied to a photobioreactor depending on positions or depths in the photobioreactor. A light intensity at a position farthest from a light source, i.e., the deepest position, in the photobioreactor is attenuated. For example, such a phenomenon is similar to a phenomenon in which the amount of light transmitted by algae located in the bottom of the ocean is reduced due to blocking of light by the top of the ocean. This is referred to self-shading effects and, in order to avoid photoinhibition caused by self-shading, proper light transmission and uniform light distribution are required.

A light source (for example, sunlight) directly affects the final yield of biomass and synthesis and accumulation of carbohydrates in algae cells, and uniform distribution of light is required to provide the same light intensity to all the algae cells.

The embodiments of the present invention, as described above, uniformly distribute light to all cells, avoid photoinhibition caused by self-shading, and provide a light wavelength band necessary depending on the growth step of the microalgae, the species of the microalgae, and a target material to be produced from the microalgae.

FIGs. 2(A) to 2(D) are views showing types of reaction units in which microalgae are cultured, according to one embodiment of the present invention.

All of the reaction units shown in FIGs. 2(A) to 2(D) are formed in a closed type. In a closed-type reaction unit, inner environments of the reaction unit, such as injection of carbon dioxide or nutrients as necessary, may be controlled, and thus, the closed-type reaction unit may have high microalgae culture efficiency. Further, the inside of the closed-type reaction unit is blocked from the outside, and thus, the closed-type reaction unit may prevent contaminants from entering the inside of the closed-type reaction unit.

FIG. 2(A) shows a column-type reaction unit and, among kinds of reaction units, the column-type will be representatively described below, and thus, a detailed description thereof will be omitted now.

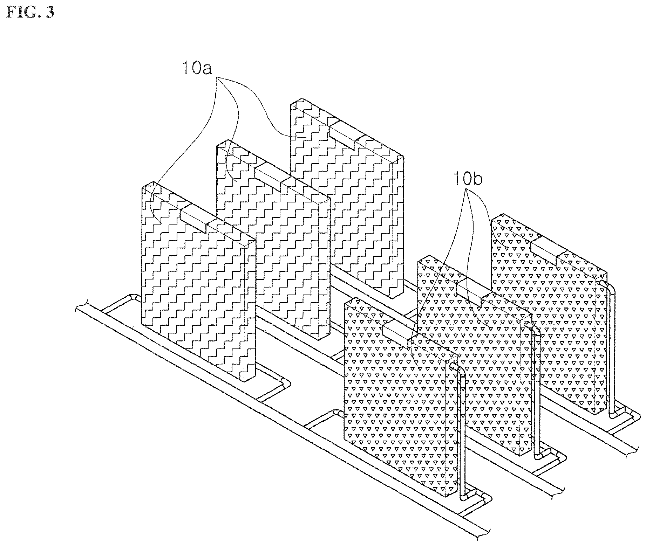

As shown in FIGs. 2(B) and 3, plate-type reaction units 10a and 10b may be formed in a rectangular or polygonal plate type having a designated area.

Here, a plurality of plate-type reaction units 10a and 10b may be disposed in a lateral direction and a longitudinal direction, and the plate-type reaction units 10a and 10b may be classified into a plurality of culture sectors in the lateral direction or the longitudinal direction. For example, a first culture sector may include first reaction units 10a, and a second culture sector may include second reaction units 10b.

The reason why the plurality of culture sectors is provided is to accommodate microalgae in different culture sectors depending on the species, the growth steps and the health statuses of the microalgae, and target materials to be produced from the microalgae and to provide different light wavelengths to the respective culture sectors. A detailed description thereof will be given below.

For reference, the respective reaction units 10a and 10b may be connected to the neighboring reaction units 10a and 10b through a transmission pipe (not numbered in the drawings), and the respective culture sectors may be connected to the neighboring culture sectors through a transmission pipe. A control valve (not shown) configured to control fluid flow may be provided on the transmission pipe.

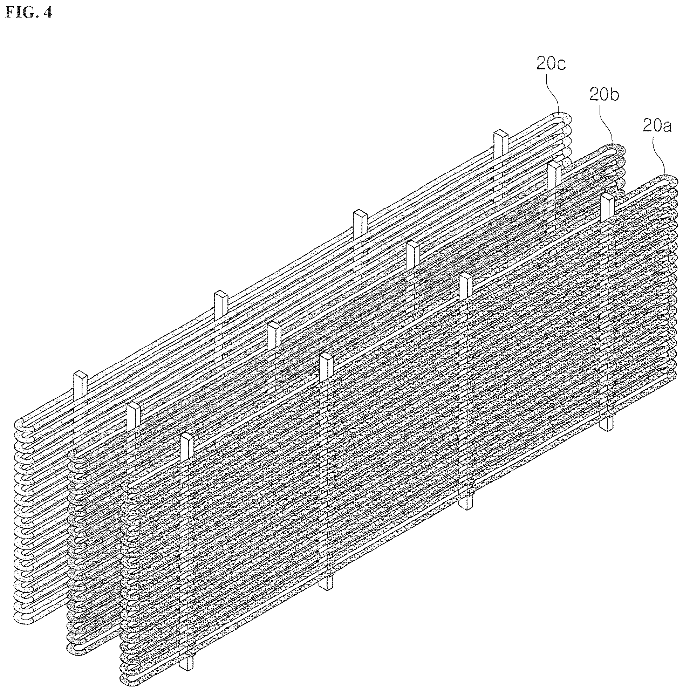

As shown in FIGs. 2(C) and 4, annular reaction units may be formed to have a designated length, and may be configured such that one end or both ends of each of the annular reaction units are bent into an annular type.

A plurality of the annular reaction units may be disposed in a direction horizontal with the ground, and may be stacked to have a plurality of layers. Each of the stacks of the annular reaction units may form one culture sector 20a, 20b or 20c, and the number of the culture sectors is not limited.

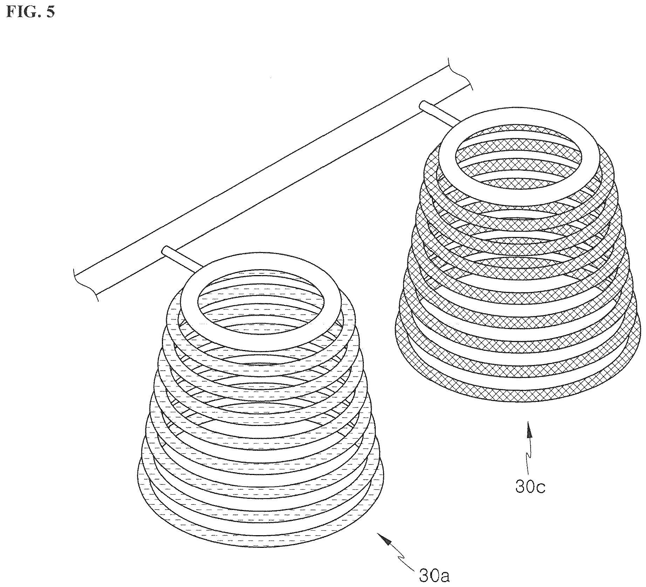

As shown in FIGs. 2(D) and 5, tubular reaction units may be provided. The tubular reaction units may be formed in a circular shape, both ends of which are connected. A plurality of the tubular reaction units may be stacked to have a plurality of layers. Each of the stacks of the tubular reaction units may form one culture sector 30a or 30b, and the number of the culture sectors is not limited.

In addition, reaction units may be formed in a sack type (not shown) having a similar shape to a bag (or a pouch), and may be formed in any types as long as the reaction units may be provided in a closed type so as not to come into contact with the outside.

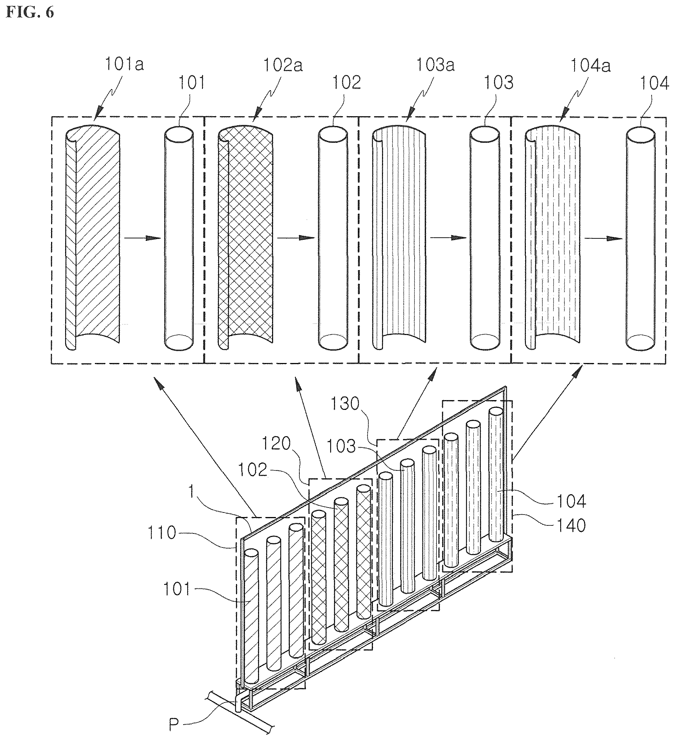

FIG. 6 is a schematic view illustrating a microalgae culture system according to one embodiment of the present invention.

As shown in FIG. 6, the microalgae culture system according to the present invention includes a plurality of culture sectors 110, 120, 130 and 140, and a control pump (not shown).

At least two culture sectors may be provided and, in this embodiment, the four culture sectors 110, 120, 130 and 140 are exemplarily provided because four growth steps of microalgae will be exemplarily described. The number of culture sectors may be changed depending on the number of the growth steps of microalgae. Further, the culture sectors 110, 120, 130 and 140 may accommodate different kinds of microalgae, and the number of culture sectors may be changed depending on the number of kinds of microalgae.

Hereinafter, the culture sectors 110, 120, 130 and 140 will be respectively defined as a first culture sector 110, a second culture sector 120, a third culture sector 130, and a fourth culture sector 140, for convenience of description.

The first culture sector 110 includes microalgae reaction units 101 using a wavelength conversion material configured to transmit light of a first wavelength. The second culture sector 120 includes microalgae reaction units 102 using a wavelength conversion material configured to transmit light of a second wavelength. The third culture sector 130 includes microalgae reaction units 103 using a wavelength conversion material configured to transmit light of a third wavelength. The fourth culture sector 140 includes microalgae reaction units 104 using a wavelength conversion material configured to transmit light of a fourth wavelength.

The respective culture sectors 110, 120, 130 and 140 may include the corresponding microalgae reaction units 101, 102, 103 and 104, and the respective microalgae reaction units 101, 102, 103 and 104 accommodate microalgae and a culture medium. Here, the culture sectors 110, 120, 130 and 140 may accommodate different species of microalgae, i.e., different targets to be cultured, may accommodate microalgae in different growth steps, may accommodate microalgae having different health statuses, or may accommodate microalgae depending on different target materials to be produced from the microalgae.

For example, the first culture sector 110 may accommodate the microalgae which is in growth step 1, the second culture sector 120 may accommodate the microalgae which is in growth step 2, the third culture sector 130 may accommodate the microalgae which is in growth step 3, and the fourth culture sector 140 may accommodate the microalgae which is in growth step 4.

For reference, a target material to be produced from the microalgae may be astaxanthin. For example, 55.1 mg/L of astaxanthin may be produced when the ratio of red light to blue light of sunlight supplied to the microalgae are adjusted to 1:3, 50.3 mg/L of astaxanthin may be produced when the ratio of red light to blue light is adjusted to 2:2, and 36.3 mg/L of astaxanthin may be produced when the ratio of red light to blue light is adjusted to 3:1. That is, the respective culture sectors 110, 120, 130 and 140 may accommodate microalgae for producing different target materials, and the outputs of the target materials to be produced may be improved by radiating different light wavelength bands to the respective culture sectors 110, 120, 130 and 140.

The reaction units 101, 102, 103 and 104 may be formed in the same shapes in the corresponding culture sectors 110, 120, 130 and 140, and the reaction units 101 included in the first culture sector 110 will be representatively described.

The reaction units 101 may be formed in a column type. For reference, the reaction units 101 may be formed in a plate type, an annular type, a tubular type, or a sack type, as described above.

The column-type reaction units 101 are disposed in a direction vertical to the ground, and the upper parts or the lower parts of the column-type reaction units 101 are supported by a support frame 1.

A plurality of the reaction units 101 may be arranged in one direction by the support frame 1. Here, each of the culture sectors 110, 120, 130 and 140 may include a predetermined number of the corresponding reaction units 101, 102, 103 or 104. For example, each of the culture sectors 110, 120, 130 and 140 may include three reaction units 101, 102, 103 or 104.

Each of the reaction units 101 may include a light-transmitting shape layer, and a wavelength conversion layer including a wavelength conversion material configured to transmit light of a predetermined wavelength. For reference, the wavelength conversion layers of the respective reaction units 101, 102, 103 and 104 may include wavelength conversion materials configured to transmit light of different wavelength bands depending on the respective culture sectors 110, 120, 130 and 140 including the reaction units 101, 102, 103 and 104. FIG. 6 exemplarily illustrates the wavelength conversion layers provided in a film type.

The light-transmitting shape layer of the reaction unit 101 may be formed of any one of polyethylene (PE), polypropylene (PP), polystyrene (PS), polyethylene terephthalate (PET), polyvinyl chloride (PVC), glass, etc. Both the upper and lower parts of the reaction unit 101 may be fixed to the support frame 1, or one of the upper and lower parts of the reaction unit 101 may be fixed to the support frame 1, depending on the material of the shape layer of the reaction unit 101. For example, when the reaction unit 101 is formed of polyvinyl chloride (PVC), only the upper part of the reaction unit 101 may be fixed to the support frame 1, or both the upper and lower parts of the reaction unit 101 may be fixed to the support frame 1.

The wavelength conversion layer of the reaction unit 101 may include a film layer 101a and a bonding layer.

The film layer 101a includes the wavelength conversion material, and is formed of a flexible material. The wavelength conversion material of the film layer 101a may include at least one of an inorganic phosphor, quantum dots or perovskite.

When light (for example, sunlight) is radiated to quantum dots included the film layer 101a, the quantum dots emit specific light depending on the size or shape of particles and the material thereof. That is, the film layer 101a converts light incident thereupon into various wavelength bands using the quantum dots, and emits the converted wavelength band.

For example, the film layer 101a may emit at least one of red light, blue light or green light through the quantum dots. For example, the film layer 101a may emit light having any one of a first wavelength band, a second wavelength band, a third wavelength band and a fourth wavelength band depending on the growth step through the quantum dots, as described above. The reaction unit 101 may include the film layer 101a configured to emit light having any one of the first wavelength band to the fourth wavelength band depending on the species or the health status of the microalgae received in the reaction unit 101 or the target material to be produced from the microalgae.

The film layers 101a of the reaction units 101 of the first culture sector 110 may include quantum dots which transmit the first wavelength band, the film layers 102a of the reaction units 102 of the second culture sector 120 may include quantum dots which transmit the second wavelength band, the film layers 103a of the reaction units 103 of the third culture sector 130 may include quantum dots which transmit the third wavelength band, and the film layers 104a of the reaction units 104 of the fourth culture sector 140 may include quantum dots which transmit the fourth wavelength band.

The bonding layer serves to bond the wavelength conversion layer to the shape layer of the reaction unit 101. An adhesive material is provided on one surface of the bonding layer.

As one example, as the wavelength conversion layer of the reaction unit 101, the above-described wavelength conversion material may be provided in the form of a paint or a pigment, and may be applied to the surface of the shape layer of the reaction unit 101, instead of the film layer 101a and the bonding layer.

As another example, the reaction unit 101 may be manufactured by performing molding of a mixture including a wavelength conversion material configured to perform wavelength conversion of a predetermined wavelength during a manufacturing process. For example, when a composite for manufacturing the reaction unit 101 includes any one of polyethylene (PE), polypropylene (PP), polystyrene (PS), polyethylene terephthalate (PET), polyvinyl chloride (PVC), glass, etc., the reaction unit 101 may be manufactured by mixing the wavelength conversion material configured to transmit light of the predetermined wavelength band with the corresponding composite.

The first culture sector 110 to the fourth culture sector 140 may be connected by a transmission pipe P. A control valve (not shown) configured to control fluid flow may be provided at a predetermined position of the transmission pipe P. The control pump is provided at one side of the transmission pipe P.

There is an order of movement of the microalgae among the culture sectors 110, 120, 130 and 140, and concretely, the microalgae accommodated in the reaction units 103 of the third culture sector 130 is moved to the reaction units 104 of the fourth culture sector 140 through the transmission pipe P by operation of the control pump. Subsequently, the microalgae accommodated in the reaction units 102 of the second culture sector 120 is moved to the reaction units 103 of the third culture sector 130 through the transmission pipe P by operation of the control pump. Next, the microalgae accommodated in the reaction units 101 of the first culture sector 110 is moved to the reaction units 102 of the second culture sector 120 through the transmission pipe P by operation of the control pump.

For reference, a bumper sector (not shown) including a bumper container (not shown) may be connected to the reaction units 104 of the fourth culture sector 140. For example, the microalgae accommodated in the reaction units 104 of the fourth culture sector 140 are moved to the bumper container of the bumper sector, before the microalgae accommodated in the reaction units 103 of the third culture sector 130 are moved to the fourth culture sector 140, and thus, the reaction units 104 of the fourth culture sector 140 may be kept vacant until the microalgae are moved thereto from the reaction units 103 of the third culture sector 130.

The control pump may be operated in a predetermined period, may be operated at manager's instructions, or may be operated by an automated algorithm.

The support frame 1 supports the plurality of reaction units 101, 102, 103 and 104. The reaction units 101, 102, 103 and 104 supported by the support frame 1 may be divided into the plurality of culture sectors 110, 120, 130 and 140.

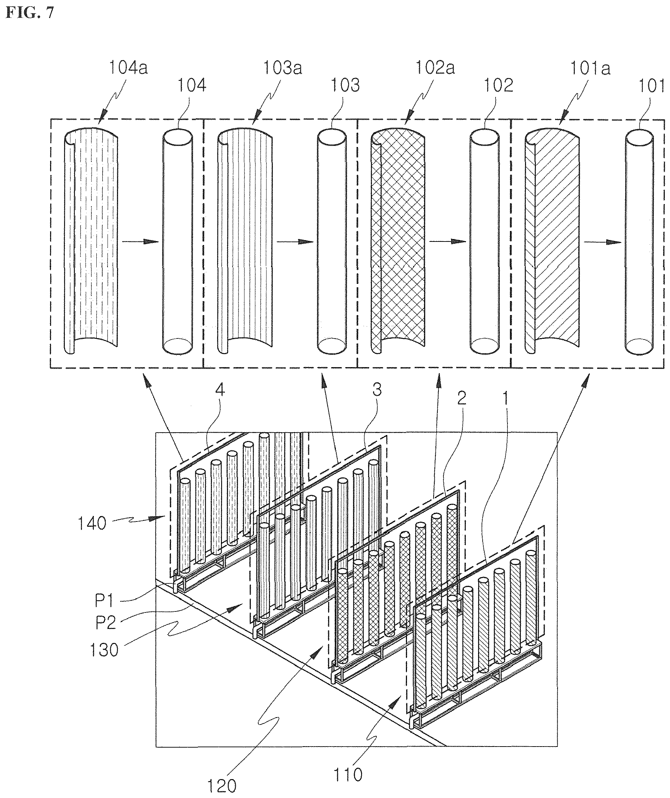

For example, when a plurality of support frames 1, 2, 3 and 4 is provided, as shown in FIG. 7, the plurality of reaction units 101, 102, 103 and 104 may be supported by the respective support frames 1, 2, 3 and 4, and may thus be divided into the culture sectors 110, 120, 130 and 140. Here, a plurality of first transmission pipes P1 is provided such that one first transmission pipe P1 connects the reaction units 101, 102, 103 or 104 included in the same culture sector 110, 120, 130 or 140, and a second transmission pipe P2 connects the respective first transmission pipes P1.

When the control pump is operated, the microalgae are moved among the culture sectors 110, 120, 130 and 140 through the first transmission pipes P1 and the second transmission pipe P2.

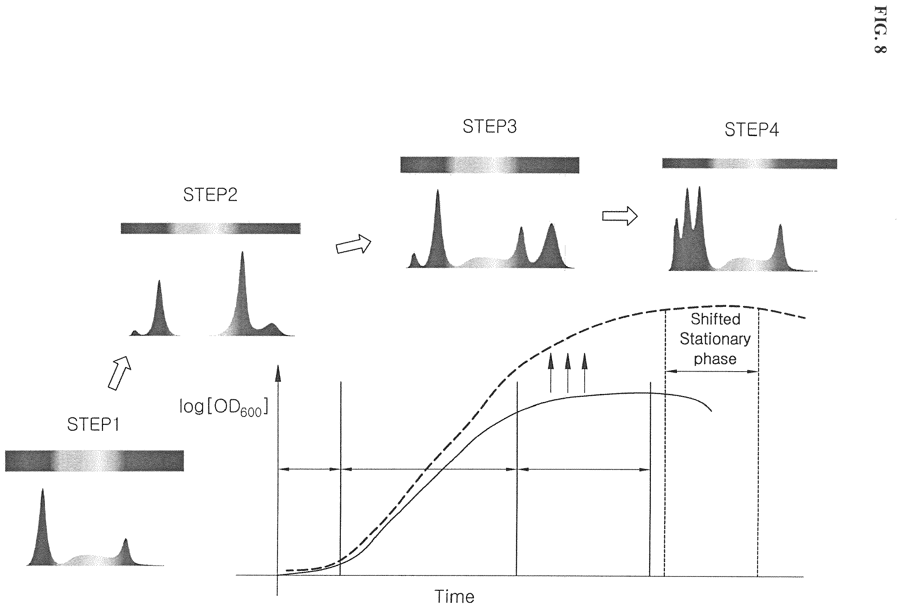

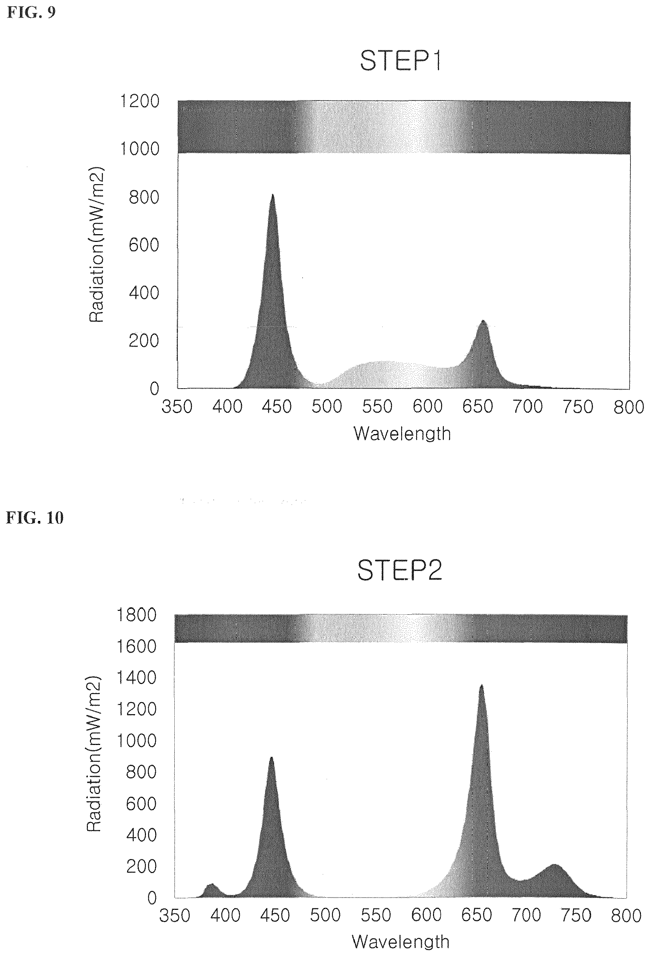

FIG. 8 is a view showing light wavelength bands provided in order of the growth steps of the microalgae in the system according to the present invention, and FIGs. 9 to 12 are graphs showing optical spectra of the light wavelength bands provided in the respective growth steps.

In one embodiment, growth of the microalgae may be classified into four steps (growth steps 1 to 4).

A light wavelength band for initial adaptation is provided to the microalgae in growth step 1, a light wavelength band for improvement in productivity of biomass is provided to the microalgae in growth step 2, a light wavelength band for prevention of photoinhibition caused by self-shading is provided to the microalgae in growth step 3, and a light wavelength band for reinforcement of a specific component/pigment is provided to the microalgae in growth step 4.

The light wavelength bands in the respective growth steps are provided by converting incident light into various light wavelength bands using the wavelength conversion material formed on the reaction units of the above-described respective culture sectors, i.e., quantum dots.

As shown in FIGs. 8 and 9, in growth step 1 of the microalgae, a proper light wavelength band to avoid the microalgae from light shock and to shorten a delay stage is provided to the microalgae so as to initially adapt the microalgae to light.

In growth step 1, a light wavelength band of 400 to 700 nm may be provided, and more concretely, a blue light wavelength band of 450 to 470 nm, a green light wavelength band of 530 to 560 nm, and a red light wavelength band of 650 to 670 nm may be provided.

As shown in FIGs. 8 and 10, in growth step 2 of the microalgae, a light wavelength band to improve productivity of the biomass is provided to the microalgae. The light wavelength band provided in this step has an important role in the growth rate and metabolism of the microalgae.

It is proved that microalgae received a blue light wavelength band have a greater cell size than that of microalgae received a red light wavelength band in growth step 2 due to delay in a cell division process.

In growth step 2, a light wavelength band of 350 to 800 nm may be provided, and more concretely, a UV light wavelength band of 350 to 400 nm, a blue light wavelength band of 450 to 470 nm, a red light wavelength band of 650 to 670 nm, and a far-red light wavelength band of 710 to 750 nm may be provided.

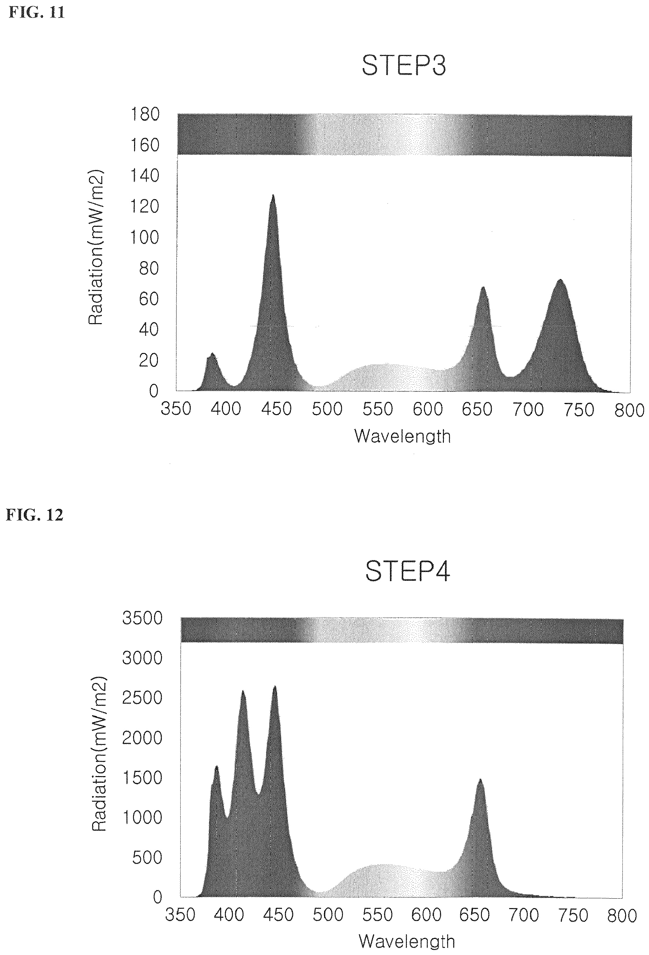

As shown in FIGs. 8 and 11, in growth step 3 of the microalgae, a light wavelength band to prevent photoinhibition caused by self-shading is provided to the microalgae.

In growth step 3, the amount of light penetrating the microalgae located in the bottom of the reaction unit may be attenuated by self-shading caused by the microalgae located in the top of the reaction unit due to growth of the microalgae. In growth step 3, in order to prevent such self-shading, a light wavelength band which may penetrate the inside of the reaction unit deeply is provided to prevent self-shading.

In growth step 3, a light wavelength band of 350 to 800 nm may be provided, and more concretely, a UV light wavelength band of 350 to 400 nm, a blue light wavelength band of 450 to 470 nm, a green light wavelength band of 530 to 560 nm, a red light wavelength band of 650 to 670 nm, and a far-red light wavelength band of 710 to 750 nm may be provided.

In growth step 4, a light wavelength band to reinforce the specific component/pigment of the microalgae are provided to the microalgae.

For example, green algae have chlorophyll a and chlorophyll b at the ratio of 3:1. Chlorophyll a has absorption peaks at 430 nm (blue/purple light) and 660 nm (deep red light), and chlorophyll b has absorption peaks at 460 nm (blue light) and 630 nm (red light).

That is, in growth step 4, a light wavelength band to reinforce the pigment which the microalgae have is provided, thereby being capable of improving production efficiency of the target material to be produced from the microalgae.

For example, in the case in which the microalgae are *Haematococcus pluvialis* and the target material to be produced is astaxanthin, a high concentration of astaxanthin may be acquired when a blue light wavelength band is provided in growth step 4.

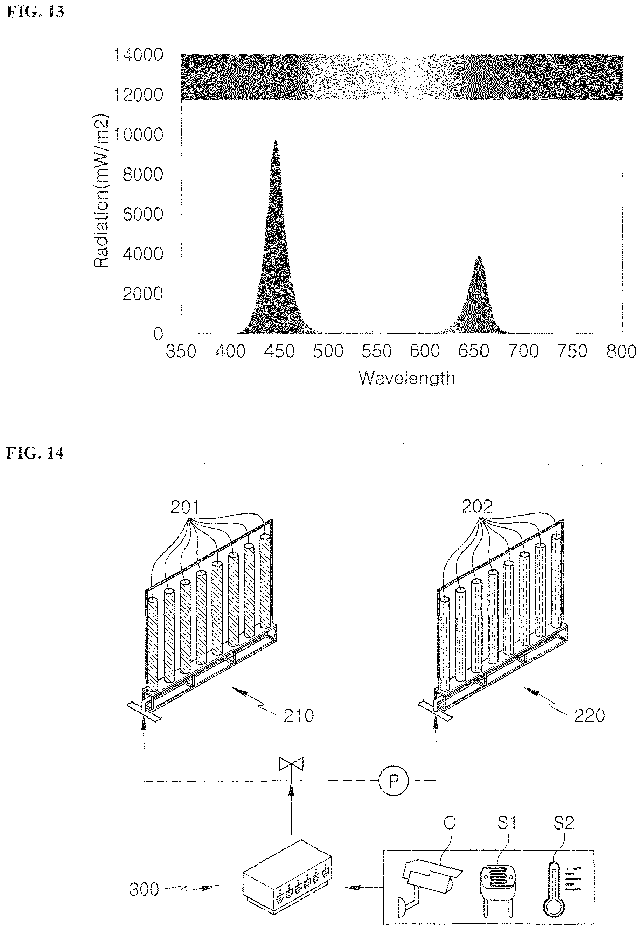

FIG. 13 is a graph representing relations between growth of the microalgae and production of astaxanthin depending on a light wavelength band.

As shown in FIG. 13, it may be provided that, in the case in which the microalgae are *Haematococcus pluvialis,* 55.1 mg/L of astaxanthin is produced when the ratio of red light to blue light is 1:3, 50.3 mg/L of astaxanthin is produced when the ratio of red light to blue light is 2:2, and 36.3 mg/L of astaxanthin is produced when the ratio of red light to blue light is 3:1. Further, the maximum biomass concentration is 1.48 g/L when the ratio of red light to blue light is 1:3, the maximum biomass concentration is 1.36 g/L when the ratio of red light to blue light is 2:2, and the maximum biomass concentration is 1.06 g/L when the ratio of red light to blue light is 3:1.

FIG. 14 is a schematic view illustrating a microalgae culture system using wavelength conversion according to another embodiment of the present invention.

Referring to FIG. 14, the microalgae culture system includes a plurality of culture sectors, each of which includes a plurality reaction units, a control pump P and a controller 300. Further, the microalgae culture system includes at least one of cameras C, optical sensors S1 or temperature sensors S2.

The reaction units, the culture sectors and the control pump of this embodiment are the same as those of the earlier embodiment and a detailed description thereof will thus be omitted, and the cameras C, the optical sensors S1, the temperature sensor S2 and the controller 300 will be described below.

Although this embodiment exemplarily describes that a first culture sector 210 and a second culture sector 220 are provided, the number of the culture sectors is not limited.

The cameras C capture the images of the reaction units which accommodate microalgae. The cameras C may be installed in a designated number so as to capture the close-up images of the respective reaction units (for example, so as to correspond to the number of the reaction units), or may be installed in a designated number so as to capture the images of the respective culture sectors (for example, so as to correspond to the number of the culture sectors). Particularly, the number of the cameras C may be the same as the number of the culture sectors, or one camera C may be installed, so as to reduce camera installation cost.

The optical sensors S1 sense the light transmittance of the respective reaction units.

The temperature sensors S2 measure the inner temperatures of the respective reaction units.

The controller 300 determines the cultured states of the microalgae in the reaction units based on at least one of the images of the reaction units captured by the cameras C, the light transmittances of the reaction units, or the inner temperatures of the reaction units.

For example, the controller 300 determines the colors of the insides of the reaction units based on the images of the reaction units captured by the cameras C, and transmits an order to transfer the microalgae to the control pump P based on input of manager's instructions or an automated algorithm, when the colors of the insides of the reaction units conform to a predetermined standard.

Here, the predetermined standard may include growth step change of the microalgae, the health status of the microalgae, or the like. For example, the controller 300 determines that the microalgae in the reaction units included in the first culture sector 210 are grown when the colors of the insides of the corresponding reaction units are changed based on the images of the reaction units captured by the cameras C, and controls the control pump P to transfer the microalgae in the reaction units included in the first culture sector 210 to the reaction units included in the second culture sector 220.

For reference, in the case in which a control valve is provided in a transmission pipe configured to transfer the microalgae from the first culture sector 210 to the second culture sector 220 therethrough, the controller 300 may control operation of the control valve in the state of being interlocked with the control pump.

As the predetermined standard to transfer the microalgae in the reaction units included in the first culture sector 210 to the insides of the reaction units included in the second culture sector 220, the controller 300 may determine that the microalgae in the reaction units included in the first culture sector 210 are grown when the light transmittances of the corresponding reaction units measured by the optical sensors S1 are changed. Further, the controller 300 may determine that the microalgae in the reaction units included in the first culture sector 210 are grown when the inner temperatures of the corresponding reaction units measured by the temperature sensors S2 are changed.

Further, a culture medium supplier (not shown) configured to supply a culture medium used to culture the microalgae may be connected to the reaction units included in the first culture sector 210 and the second culture sector 220.

The culture medium supplier may be controlled by the controller 300.

The controller 300 supplies the culture medium to the reaction units which conform to the predetermined standard. For example, the predetermined standard for controlling the culture medium supplier by the controller 300 may be change in the colors of the insides of the reaction units based on the images of the reaction units captured by the cameras C, change in the light transmittances of the reaction units measured by the optical sensors S1, or change in the inner temperatures of the reaction units measured by the temperature sensors S2.

For example, the controller 300 may determine that the microalgae in the reaction unit is grown when the color value of the inside of the corresponding reaction unit becomes +1, and may determine that the culture medium in the reaction unit is insufficient when the color value of the inside of the corresponding reaction unit becomes -1.

The present invention was devised during execution of the following R & D project.
- PROJECT ID: 1545024479
- PROJECT NO.: 421008-04
- GOVERNMENT DEPARTMENT: The Ministry of Agriculture, Food and Rural Affairs, The Ministry of Science and ICT, and The Rural Development Administration
- RESEARCH AND MANAGEMENT INSTITUTE: The Korea Institute of Planning and Evaluation for Technology in Food Agriculture and Forestry, Korea Smart Farm R&D Foundation
- RESEARCH PROJECT NAME: Smart Farm Multi-Ministry Package Innovative Technology Development
- RESEARCH SUBJECT NAME: Development of High Temperature and Humidity-type Smart Greenhouse Package Model for Export
- CONTRIBUTION RATE: 1/1
- PROJECT EXECUTION ORGANIZATION: Sherpa Space Inc.
- RESEARCH PERIOD: 2020. 1. 1 to 2022. 12. 31

As is apparent from the above description, the embodiments of the present invention provide a microalgae reaction unit using wavelength conversion which selectively provides light having a predetermined wavelength band depending on the species, the growth step and the health status of microalgae received in the reaction unit and a target material to be produced from the microalgae, so as to increase growth efficiency of the microalgae or to improve the output of the target material to be produced from the microalgae, and a microalgae culture system using the microalgae reaction unit.

Although the exemplary embodiments of the present invention have been disclosed for illustrative purposes, those skilled in the art will appreciate that various modifications, additions and substitutions are possible, without departing from the scope and spirit of the invention as disclosed in the accompanying claims.

## Claims

1. A microalgae culture system using wavelength conversion, comprising:
a first culture sector comprising microalgae reaction units using a wavelength conversion material configured to transmit light of a first wavelength;
a second culture sector comprising microalgae reaction units using a wavelength conversion material configured to transmit light of a second wavelength; and
a control pump configured to transfer microalgae in the first culture sector to the second culture sector through a transmission pipe, when a control condition is achieved.

2. The microalgae culture system according to claim 1, wherein the first wavelength and the second wavelength are determined depending on at least one of a species of the microalgae corresponding to a target to be cultured, a growth step of the microalgae, a health status of the microalgae, or a target material to be produced from the microalgae.

3. The microalgae culture system according to claim 1 or 2, wherein the microalgae reaction units are closed-type units having any one of a plate type, a column type, an annular type, a tubular type, and a sack type.

4. The microalgae culture system according to any of the preceding claims, wherein each of the microalgae reaction units comprises a light-transmitting shape layer, and a wavelength conversion layer comprising a wavelength conversion material configured to transmit light of a predetermined wavelength band.

5. The microalgae culture system according to claim 4, wherein the wavelength conversion layer comprises a flexible film layer comprising the wavelength conversion material, and a bonding layer having adhesiveness to be adhered to the shape layer.

6. The microalgae culture system according to claim 4 or 5, wherein the wavelength conversion layer is formed by applying the wavelength conversion material to the shape layer.

7. The microalgae culture system according to claim 3, wherein the microalgae reaction units are manufactured by performing molding of a mixture of a light-transmitting shape material and a wavelength conversion material configured to transmit light of a predetermined wavelength band.

8. The microalgae culture system according to any of the preceding claims, further comprising:
at least one of cameras configured to capture images of the microalgae reaction units, optical sensors configured to sense light transmittances of the microalgae reaction units, or temperature sensors configured to measure inner temperatures of the microalgae reaction units; and
a controller configured to determine cultured states of the microalgae in the microalgae reaction units based on at least one of the images captured by the cameras, the light transmittances, or the inner temperatures, and to transmit light of an order to transfer the microalgae to the control pump based on input of manager's instructions or an automated algorithm when the determined cultured states of the microalgae conform to a predetermined standard.

9. The microalgae culture system according to any of the preceding claims, wherein the wavelength conversion material comprises at least one of an inorganic phosphor, quantum dots or perovskite.

10. A microalgae reaction unit using wavelength conversion, configured to employ a wavelength conversion material configured to selectively transmit light of a predetermined wavelength band depending on at least one of a species microalgae corresponding to a target to be cultured, a growth step of the microalgae, a health status of the microalgae, or a target material to be produced from the microalgae.

11. The microalgae reaction unit according to claim 10, configured to be a closed-type unit having any one of a plate type, a column type, an annular type, a tubular type, and a sack type.

12. The microalgae reaction unit according to claim 10 or 11, comprising a light-transmitting shape layer, and a wavelength conversion layer comprising a wavelength conversion material configured to transmit light of a predetermined wavelength band,
particularly wherein the wavelength conversion layer comprises a flexible film layer comprising the wavelength conversion material, and a bonding layer having adhesiveness to be adhered to the shape layer.

13. The microalgae reaction unit according to claim 12, wherein the wavelength conversion layer is formed by applying the wavelength conversion material to the shape layer.

14. The microalgae reaction unit according to claim 11, manufactured by performing molding of a mixture of a light-transmitting shape material and a wavelength conversion material configured to transmit light of a predetermined wavelength band.

15. The microalgae reaction unit according to any of claims 10-14, wherein the wavelength conversion material comprises at least one of an inorganic phosphor, quantum dots or perovskite.
